# EUROPEAN PATENT SPECIFICATION

(11) **EP 3 076 861 B1**
(45) Date of publication and mention of the grant of the patent: **20.12.2017**
(21) Application number: 15760176.6
(22) Date of filing: 08.09.2015
(51) Int. Cl.: A61B 5/00, A61B 5/024, A61B 5/026

(54) **VITAL SIGNS MONITORING SYSTEM**
ÜBERWACHUNGSSYSTEM FÜR LEBENSFUNKTIONEN
SYSTÈME DE SURVEILLANCE DES SIGNES VITAUX

(30) Priority: 09.09.2014 EP 14184098
(43) Date of publication of application: 12.10.2016
(73) Proprietor: Koninklijke Philips N.V., 5656 AE Eindhoven (NL)
(72) Inventor: PRESURA, Cristian Nicolae, NL-5656 AE Eindhoven (NL); PAULUSSEN, Elvira Johanna Maria, NL-5656 AE Eindhoven (NL); VAN PUTTEN, Eibert Gerjan, NL-5656 AE Eindhoven (NL); VERMEULEN, Olaf Thomas Johan Antonie, NL-5656 AE Eindhoven (NL)
(74) Representative: Roche, Denis
(86) International application number: PCT/EP2015/070415
(87) International publication number: WO 2016/037991

(56) References cited:
- US-A1- 2003 120 156
- US-A1- 2009 259 098
- US-A1- 2010 224 796
- US-A1- 2011 319 712
- US-A1- 2012 188 354
- US-A1- 2013 169 966
- US-A1- 2014 058 226
- US-A1- 2014 200 423

## Description

### FIELD OF THE INVENTION

The invention relates to a vital signs monitoring system as well as a method of monitoring vital signs of a user.

### BACKGROUND OF THE INVENTION

Optical heart rate sensors are well known to monitor or detect vital signs like a heart rate of a user. Such a heart rate sensor can be based on a photoplethysmographic (PPG) sensor and can be used to acquire a volumetric organ measurement. By means of pulse oximeters, changes in light absorption of a human skin is detected and based on these measurements a heart rate or other vital signs of a user can be determined. The PPG sensors comprise a light source like a light emitting diode (LED) which is emitting light into the skin of a user. The emitted light is scattered in the skin and is at least partially absorbed by the blood. Part of the light exits the skin and can be captured by a photodiode. The amount of light that is captured by the photodiode can be an indication of the blood volume inside the skin of a user. A PPG sensor can monitor the perfusion of blood in the dermis and subcutaneous tissue of the skin through an absorption measurement at a specific wavelength. If the blood volume is changed due to the pulsating heart, the scattered light coming back from the skin of the user is also changing. Therefore, by monitoring the detected light signal by means of the photodiode, a pulse of a user in his skin and thus the heart rate can be determined.

US 2007/287927 discloses a vital signs measurement device with an optical sensing system. The optical sensing system comprises an optical source producing a speckle pattern output and an optical detector to detect at least part of the speckle pattern. The optical source is a coherent light source like a laser. Alternatively, an LED can be used.

US2014/200423A1 discloses a pulse oximetry device that is mounted on a wrist strap and fixates an area above a distal end of the ulna with a dome shaped structure. This area is used as measuring area. The measurement is carried out by a detector positioned above the fixated area that detects light emitted by light sources having different wave lengths that are located at a periphery of the fixated area. Hence, the reflections are measured at neither a reflection mode nor a transmission mode, but are at an angle between 20° and 160° from the emitted light. This mode, termed trans-illumination, allows achieving an excellent signal to noise ratio that for the first time enables continuous and reliable measurement of oximetry data on the wrist.

### SUMMARY OF THE INVENTION

It is an object of the invention to provide a vital signs monitoring system, which is able to efficiently detect a heart rate of a user as well as additional vital sign information.

In an aspect of the present invention, a vital sign monitoring system is provided. The vital sign monitoring system comprises at least one optical vital signs sensor configured to measure or determine vital signs of the user and to output an output signal. The optical vital signs sensor comprises at least one laser light source configured to generate laser light, which is directed towards a skin of a user. The optical vital signs sensor furthermore comprises at least one photo detector unit configured to detect light which is indicative of an absorption or reflection of the laser light from the at least one light source in or from the skin of the user. The vital signs monitoring system furthermore comprises a coherence distortion unit. In a first operational mode the coherence distortion unit is configured to distort a wave front from laser light from the at least one laser light source such that the coherence of the light that is directed towards the skin of a user is distorted. In a second operational mode the coherence distortion unit is configured to deactivate the distortion of the wave front of the laser light such that coherent light is directed undistorted towards the skin of the user. Accordingly, by means of the coherent distortion unit a single light source in the form of a laser light source can be used and the light entering the skin of the user can have two different modes, namely coherent or non-coherent. The vital signs monitoring system comprises an analyzing unit with a heart rate analyzing unit and a microcirculation analyzing unit. In the first operational mode the heart rate analyzing unit is configured to analyze the output of the at least one photo detector to determine the heart rate of a user. In a second operational mode the microcirculation analyzing unit is configured to analyze the output of the at least one photo detector to determine parameters of microcirculation. Hence, in a first operational mode the heart rate of a user can be detected and in second operational modes parameters of the microcirculation can be detected.

According to an aspect of the invention the at least one optical vital signs sensor is a photoplethysmographic PPG sensor.

In a further aspect of the invention, the vital signs monitoring system comprises a control unit for activating the first or second operational mode.

In a further aspect of the present invention, the coherence distortion is configured to control an input current supply to the at least one light source in order to change the emitting mode of the laser light source to distort the wave front of the laser light from the at least one laser light source in the first operational mode.

According to a further aspect of the invention, the coherence distortion unit comprises a phase plate or phase diffuser configured to spatially distort a phase of the wave front of the laser light from the at least one laser light source.

According to a further aspect of the invention, the coherence distortion unit comprises an electrically control diffuser to spatially distort a phase of the wave front of the laser light from the at least one laser light source.

According to a further aspect of the invention, the coherence distortion unit comprises a phase modulator to distort a phase of the wave front of the laser light from the at least one laser light source. Examples of such phase modulators are electro-optical EO modulators comprising a non-linear chrystal, acousto-optical (AO) modulators and (rotating) phase plates.

In a further aspect of the invention, the coherence distortion unit comprises a spatial light modulator to spatially distort a phase of the wave front of the laser light from the at least one laser light source i.e. by changing the diffusivity. Examples of such spatial light modulators are liquid crystal (LC) modulators, electro-chromic modulators and mechanical modulators like rotation wheels, scanning systems, or piezo- or galvano scanners.

In a further aspect of the invention, the coherence distortion unit comprises a wavelength modulator configured to change the wavelength of the light thereby distorting the wave front. Such modulators can be an etalon or Fabry-Pérot interferometer or an electro-optical modulator.

According to an aspect of the invention, the coherence distortion unit can be implemented as an optically addressed modulator (like a liquid crystal modulator, an electro-chromic modulator, an electro optical modulator, an acousto-optical modulator), as an electrically addressed modulator (like a rotating diffuser, a rotating phase plate, a galvanic or piezoelectric device) or as a wavelength modulator.

According to an aspect of the invention it was realized that the typically unwanted high frequency noise in the output of a PPG sensor (when using a laser light source) can be used to advantage if it can be discarded for the detection of the heart rate while being used to obtain information on the microcirculation of the user. Hence, speckle analysis can be used according to an aspect of the invention to detect microcirculation.

According to a further aspect of the invention, the at least one laser light source comprises a stepped ultra-thin cavity solid state laser.

It shall be understood that a preferred embodiment of the present invention can also be a combination of the dependent claims or above embodiments or aspects with respective independent claims.

These and other aspects of the invention will be apparent from and elucidated with reference to the embodiment(s) described hereinafter.

### BRIEF DESCRIPTION OF THE DRAWINGS

In the following drawings:
Fig. 1 shows a basic representation of the operational principal of vital sign monitoring system according to an aspect of the invention,
Fig. 2 shows a graph indicating an output signal of photo detector of a vital sign monitoring system according to an aspect of the invention,
Fig. 3A shows as representation of a vital sign detection in a first mode of operation with light having a distorted wave front,
Fig. 3B shows a basic representation of a vital sign detection in a second mode of operation with light having an undistorted wave front,
Fig. 4A shows a basic representation of a vital sign detection in a second operation,
Fig. 4B shows a graph indicating an output of a photo detector in a second operational mode,
Fig. 4C shows a graph indicating an enlarge portion of an output signal of a photo detector according to the invention,
Fig. 5 shows a block diagram of a vital sign monitoring system according to an aspect of the invention,
Fig. 6A and 6B each show a schematic representation of a laser light source for a vital sign monitoring system according to an aspect of the invention, and
Fig. 7 shows a schematic cross section of a vital sign monitoring system according to an aspect of the invention.

### DETAILED DESCRIPTION OF EMBODIMENTS

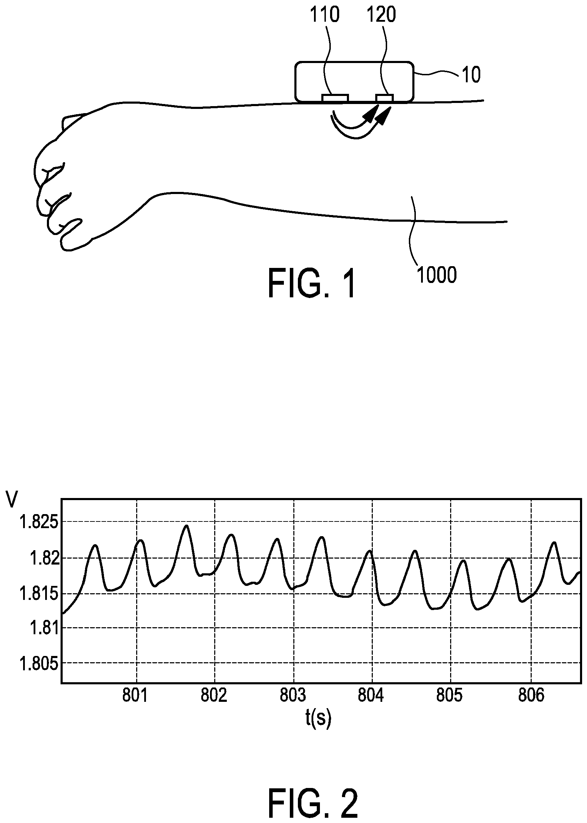

Fig. 1 shows a basic representation of the operational principal of vital sign monitoring system according an aspect of the invention. In Fig. 1 a vital sign monitoring system 10 according to an aspect of the invention is disclosed on an arm of a user. The vital sign monitoring system 10 can be embodied as a heart rate sensor, which comprises a laser light source 110 and a photo detector 120. The laser light source 110 emits light onto or in the skin 1000 of a user. Here, some of the light is reflected and the reflected light can be detected by the photo detector 120.

Fig. 2 shows a graph indicating an output signal of a photo detector for a vital sign monitoring system according to an aspect of the invention. In particular, in Fig. 2 the time as well as the output voltage of a photo detector 120 is depicted. As can be seen, the peaks in the output voltage V can correspond to the pulse of the user and thereby the heart rate of a user can be determined.

According to the invention, the light source 110 can be implemented as a laser light source. When the heart of a user pulsates, this results in a changing blood volume in the skin of the user which can be detected by the photo detector 120. According to the invention, the laser light source 110 can be operated in a first and second operational mode. In first operational mode, a coherence disturbance unit can be activated in order to disturb or distort a wave front of the output signal of the laser light source. In a second operational mode, the coherence distortion unit can be deactivated such that the laser light source is able to emit coherent light. In the first operational mode, the operational mode only non-coherent light is emitted onto or into the skin of the user.

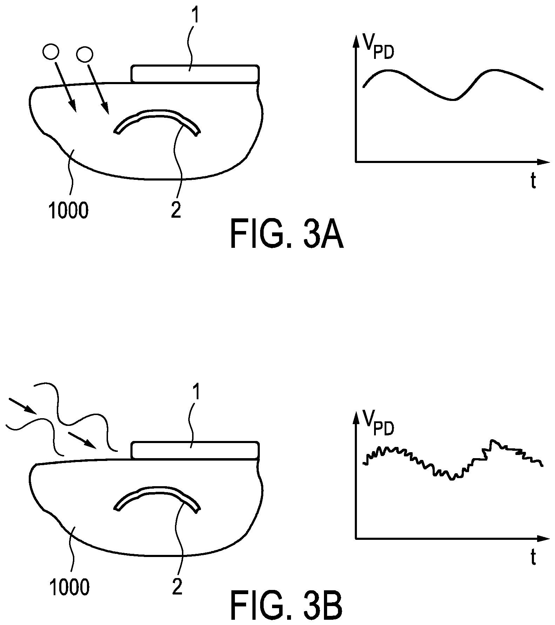

Fig. 3A shows as representation of a vital sign detection in a first mode of operation with light having a distorted wave front. Fig. 3B shows a basic representation of a vital sign detection in a second mode of operation with light having an undistorted wave front. The light is emitted into a skin 1000 of a user, interacts with moving blood 2 and is detected by a camera 1 like an image camera.

In Fig. 3A the operation of the vital sign monitoring system in a first operational mode is depicted. Here, the coherence distortion is activated such that non-coherent light enters the skin 1000 of a user and can interact with blood inside of the skin. In the first operational mode as shown in Fig. 3A, the light emitted by the laser light source substantially corresponds to light emitted by a light emitting diode. In Fig. 3A the output signal V_{PD} of the photo detector 120 is depicted over time t.

In Fig. 3B the operation of the vital sign monitoring system according in a second operational mode is depicted. Here, the coherence distortion unit is deactivated such that coherent light (i.e. with an undistorted wave front) from the laser light source is entering the skin 1000 of the user and interacts with the blood 2. The output signal V_{PD} of the photo detector 120 is also depicted in Fig. 3B. Here, it can be seen that a ripple is present on the output signal. In particular, because of the Doppler effect a high frequent ripple is present in the output signal of the photo detector 120. This high frequent ripple in the output signal of the photo detector is a result of laser speckle. The presence of the high frequent ripple makes the detection of the heart rate a lot more difficult. Therefore, typically for detecting a heart rate of a user it is not preferable to use a laser light source.

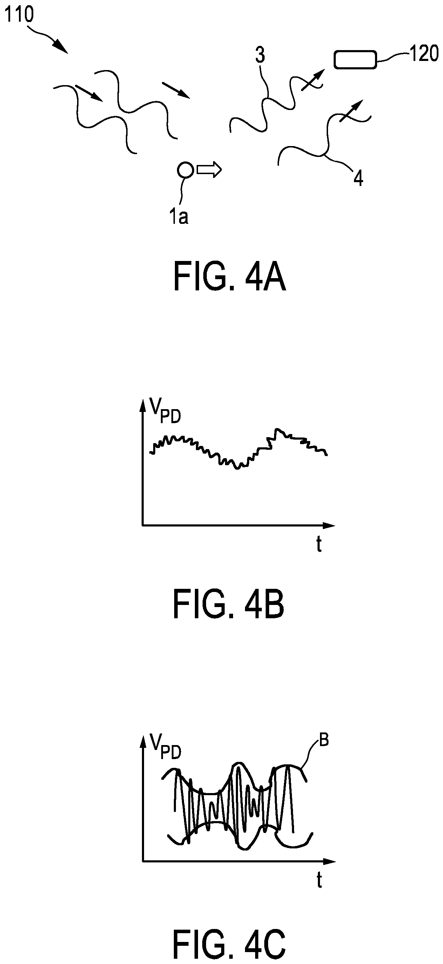

In the Fig. 4A-4C the effect of the usage of a laser light source with coherent light is depicted. As the coherent light from the laser light source enters the skin of the user and interacts with moving blood cells 1a, a part of the signal is reflected, wherein the reflected signal will contain a part including Doppler frequency shift 3 as well as a not shifted component 4. In Fig. 4B and 4C the output signal V_{PD} of the photo detector 120 is depicted over time t. In Fig. 4C heart beats B can be detected. The result of that is depicted in Fig. 4B, where the high frequent ripple can be seen.

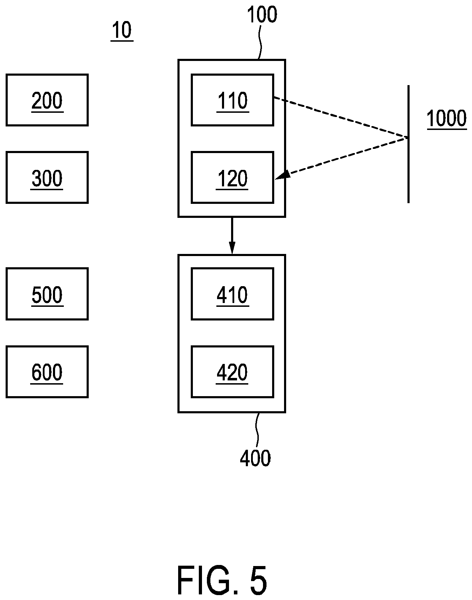

Fig. 5 shows a block diagram of vital sign monitoring system according to the invention. The vital sign monitoring system 10 can be implemented as a heart rate monitoring or detecting system. The monitoring system 10 therefore comprises an optical vital sign sensor 100, which uses a photoplethysmograph PPG sensor to detect a heart rate or other vital signs of a user. The PPG sensor 100 comprises at least one laser light source 110 and at least one photo detector 120. The light from the laser light source 110 is directed to the skin 1000 of a user. The photo detector 120 can detect reflected light.

The vital sign monitoring system 10 furthermore comprises a coherence distortion unit 200 and optionally a control unit 300. The output of the at least one photo detector 120 can be analyzed in an analyzing unit 400.

Optionally, secondary sensors 500 like accelerometers and an optionally battery unit 600 can be provided. The sensors from the secondary sensor unit 500 can be used to verify or improve the detection or determinations of vital signs.

According to an aspect of the invention the vital sign monitoring system 10 can be embodied as a wrist device for example a smart watch. In this case, the device may also comprise a battery unit 600.

The vital sign monitoring system 10 can be operated in a first and second operating mode. The control unit 300 is able to switch the vital sign monitoring system between at least first and second operating mode. In the first operating mode, the coherence distortion unit 200 is activated such that substantially non-coherent light reaches the skin 1000 of the user. By means of the coherence disturbing unit 200 the wave front of the laser light can be distorted to achieve a better signal. With the distorted and non-coherent light it is a lot easier to detect a heart rate of a user than with coherent light. Thus, by means of the coherence distortion unit 200 the wave front of the laser light is distorted in order to achieve a better signal for heart rate detection.

According an aspect of the invention, coherence disturbance unit 200 is adapted to control the electric current, which is supplied to the at least one laser light source 110. As the wavelength of emission of a semiconductor laser diode is dependent on its laser current (is e.g. used in Wavelength Modulation Spectroscopy), changes in the laser drive current will change the emitted mode. If the changes of the emitting mode occurs rapidly with frequencies of more than ten kHz the speckle pattern change can occur at a similar rate. In order to change the speckle pattern the average optical path length through the scattering medium and the average optical wavelength need to be change into achieve change of the typical wavelength. For a wavelength of 850 nm and an optical path length of approximately 5 nm, the required wavelength change is equal or larger than 70 picometer.

According to a further aspect of the invention the coherence disturbance unit 200 can be implemented by a phase plate or diffuser, which is arrange in front of the laser light source 110. The phase plate or the diffuser can revolve or rotate such that the wave front will change accordingly. For a frequency larger than several kHz, the speckle pattern will change accordingly. Hence, the phase of the wavelength can be spatially distorted by an electrical-optical cell such as a spatial light modulator.

According to a further aspect of the invention the coherence disturbance unit 200 can be implemented as an electrically controlled diffuser.

According to a further aspect of the invention, the laser light source and the coherence disturbance unit can together be implemented as a vertical-cavity surface emitting laser (VCSEL).

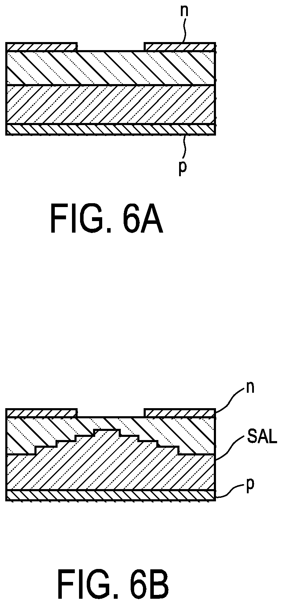

In particular the vertical-cavity surface emitting laser can have a stepped design in the cavity as shown in Fig. 6B as compared to a usual laser as depicted in Fig. 6A. By means of the stepped vertical-cavity surface emitting laser the wave front of the laser is distorted by the stepped design of the cavity. The vertical-cavity surface emitting laser comprises a n-contact n, a p-contact p and a stepped active layer (SAL) in between.

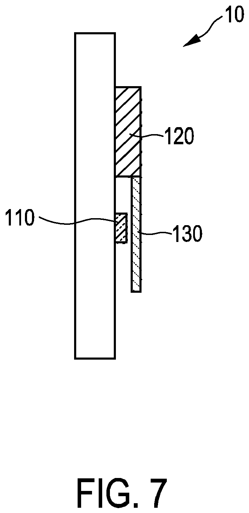

Fig. 7 shows a schematic cross section of a vital sign monitoring system according to an aspect of the invention. The optical heart rate sensor 10 comprises at least one laser light source 110, a photodiode 120 and a modulator unit 130, which is arranged for example in front of the laser source 110. The modulator unit 130 serves to distort the wave front of the light from the laser light source 110 when activated. If not activated, the light is not distorted. The modulator unit 130 can for example be implemented as a phase modulator to distort a phase of the wave front of the laser light. Alternatively, the modulator unit can be implemented as an electrically controlled diffuser, a spatial light modulator, an electro-chromic unit or a wave length modulator. The liquid crystal modulator 130 acts as a spatial light modulator such that a phase of the wave front of the laser variation is distorted by the liquid crystal in the liquid crystal modulator 130 such the coherent light from the laser light source 110 transformed to non-coherent light.

Based on the non-coherent light as well as the detected reflections, the vital signs monitoring unit can determine the heart rate of a user when operating in a first operation mode.

In a second operational mode, the coherence disturbance unit 200 is deactivated and coherent light from the laser light source enters the skin of the user. The reflected signal as detected by the photodiode 120 can be used in order to analyze microcirculation of the blood knowledge on the microcirculation in the advantageous hypertension, heart failure, hypercholesterolmea, Alzheimer disease, carpal tunnel syndrome, schizophrenia, renal type 2 diabetes, peripheral vascular disease, atherosclerotic coronary disease, systematic scleroses, obesity, primary aging, sleep apnea, wound assessment, plastic surgery, Doppler cuff blood pressure, peripheral arterial occlusive disease and edema.

According to a further aspect of the invention the coherence disturbance unit 200 can be implemented as an electro-chromic device for disturbing the wave front of the laser by changing the diffusivity of the laser light.

In an aspect of the invention the coherence distortion unit can be implemented as a phase modulator to distort the phase of the wave front. The phase modulator can be an electro-optical (EO) modulator, an AO modulator, rotating phase plate, or alternatively galvanic or piezoelectric steered devices.

Other variations of the disclosed embodiment can be understood and effected by those skilled in the art in practicing the claimed invention from a study of the drawings, the disclosure and the appended claims.

In the claims, the word "comprising" does not exclude other elements or steps and in the indefinite article "a" or "an" does not exclude a plurality.

A single unit or device may fulfill the functions of several items recited in the claims. The mere fact that certain measures are recited in mutual different dependent claims does not indicate that a combination of these measurements cannot be used to advantage. A computer program may be stored/distributed on a suitable medium such as an optical storage medium or a solid state medium, supplied together with or as a part of other hardware, but may also be distributed in other forms such as via the internet or other wired or wireless telecommunication systems.

Any reference signs in the claims should not be construed as limiting the scope.

## Claims

1. Vital signs monitoring system, comprising:
- at least one optical vital signs sensor (100) configured to measure or determine vital signs of a user and to output an output signal, wherein said at least one optical vital signs sensor (100) comprises at least one laser light source (110) configured to generate laser light, which is directed towards a skin (1000) of the user, and at least one photo detector unit (120) configured to detect light which is indicative of an absorption or reflection of the laser light from the at least one light source (100) in or from the skin (1000) of the user,
- a coherence distortion unit (200) in a first operational mode configured to distort a wave front of the laser light from the at least one laser light source (110) such that non-coherent light is directed towards the skin (1000) of the user and in a second operational mode configured to deactivate the distortion of the wave front of the laser light such that coherent light is directed towards the skin (1000) of the user, and
- an analyzing unit (400) having a heart rate analyzing unit (410) and a microcirculation analyzing unit (420) and being configured to analyze the output signal of the at least one optical vital signs sensor (100), wherein in the first operational mode the heart rate analyzing unit (410) is configured to analyze the output of the at least one photo detector (120) to determine a heart rate of the user, wherein in a second operational mode the microcirculation analyzing unit (420) is configured to analyze the output of the at least one photo detector (120) to determine parameters of microcirculation of the user.

2. Vital signs monitoring system according to claim 1, wherein the at least one optical vital signs sensor (100) is a photoplethysmographic sensor.

3. Vital signs monitoring system according to claim 2, further comprising a control unit (300) configured to activate the first or second operational mode.

4. Vital signs monitoring system according to claim 1, wherein the coherence distortion unit (200) is configured to control an input current supplied to the at least one laser light source (110) in order to distort the wave front of the laser light from the at least one laser light source (110) in the first operational mode.

5. Vital signs monitoring system according to claim 1, wherein the coherence distortion unit (200) comprises a phase modulator (130) configured to distort a phase of the wave front of the laser light from the at least one laser light source (110).

6. Vital signs monitoring system according to claim 1, wherein the coherence distortion unit (200) comprises an electrically controlled diffuser (130) to spatially distort the wave front of the laser light from the at least one laser light source (110).

7. Vital signs monitoring system according to claim 1, wherein the coherence distortion unit (200) comprises a spatial light modulator (130) to spatially distort the wave front of the laser light from the at least one laser light source (110).

8. Vital signs monitoring system according to claim 1, wherein the coherence distortion unit (200) comprises an electro-chromic or liquid crystal unit (130) to spatially distort the wave front of the laser light from the at least one laser light source (110) by changing the diffusivity.

9. Vital signs monitoring system according to claim 1, wherein the coherence distortion unit (200) comprises a wavelength modulator (130) configured to change the wavelength of the light to distort the wave front of the light from the at least one laser light source (110).

10. Vital signs monitoring system according to claim 1, wherein the at least one laser light source (110) comprises a stepped ultra-thin cavity solid state laser.

11. Method of monitoring vital signs of a user, comprising the steps of:
- measuring or determining vital signs of a user by at least one optical vital signs sensor (100), wherein said at least one optical vital signs sensor (100) comprises at least one laser light source (110) configured to generate laser light, which is directed towards a skin (1000) of a user, and at least one photo detector unit (120) configured to detect light which is indicative of an absorption or reflection of the laser light from the at least one light source (100) in or from the skin (1000) of the user, and
- in a first operational mode, distorting a wave front of the laser light from the at least one laser light source (110) such that non-coherent light is directed towards the skin (1000) of the user, and
- in a second operational mode, deactivating the distortion of the wave front of the laser light such that coherent light is directed towards the skin (1000) of the user, and
- analyzing the output signal of the at least one optical vital signs sensor (100), wherein in the first operational mode the analyzing step is adapted to analyze the output of the at least one photo detector (120) to determine a heart rate of the user, wherein in a second operational mode the analyzing step is adapted to analyze the output of the at least one photo detector (120) to determine parameters of microcirculation of the user.

12. A computer program product, comprising a computer readable memory storing computer program code means for causing a processor to carry out steps of the monitoring vital signs of a user according to claim 11.

## Patentansprüche

1. Vitalzeichenüberwachungssystem, umfassend:
- mindestens einen optischen Vitalzeichensensor (100), der so konfiguriert ist, dass er Vitalzeichen eines Benutzers misst oder ermittelt und ein Ausgangssignal ausgibt, wobei der mindestens eine optische Vitalzeichensensor (100) mindestens eine Laserlichtquelle (110), die so eingerichtet ist, dass sie Laserlicht erzeugt, das auf eine Haut (1000) des Benutzers gerichtet ist, sowie mindestens eine Photodetektoreinheit (120) umfasst, die so konfiguriert ist, dass sie Licht, das für eine Absorption oder Reflexion des Laserlichts aus der mindestens einen Lichtquelle (100) bezeichnend ist, in oder auf der Haut (1000) des Benutzers detektiert,
- eine Kohärenzverzerrungseinheit (200), die in einem ersten Betriebsmodus so konfiguriert ist, dass sie eine Wellenfront des Laserlichts aus der mindestens einen Laserlichtquelle (110) so verzerrt, dass nicht-kohärentes Licht auf die Haut (1000) des Benutzers gerichtet wird, und in einem zweiten Betriebsmodus so konfiguriert ist, dass sie die Verzerrung der Wellenfront des Laserlichts so deaktiviert, dass kohärentes Licht auf die Haut (1000) des Benutzers gerichtet wird, sowie
- eine Analysiereinheit (400), die eine Herzfrequenzanalysiereinheit (410) sowie eine Mikrozirkulationsanalysiereinheit (420) umfasst und so konfiguriert ist, dass sie das Ausgangssignal des mindestens einen optischen Vitalzeichensensors (100) analysiert, wobei in dem ersten Betriebsmodus die Herzfrequenzanalysiereinheit (410) so konfiguriert ist, dass sie die Ausgabe des mindestens einen Photodetektors (120) analysiert, um eine Herzfrequenz des Benutzers zu ermitteln, wobei in einem zweiten Betriebsmodus die Mikrozirkulationsanalysiereinheit (420) so konfiguriert ist, dass sie die Ausgabe des mindestens einen Photodetektors (120) analysiert, um Mikrozirkulationsparameter des Benutzers zu ermitteln.

2. Vitalzeichenüberwachungssystem nach Anspruch 1, wobei der mindestens eine optische Vitalzeichensensor (100) ein photoplethysmographischer Sensor ist.

3. Vitalzeichenüberwachungssystem nach Anspruch 2, weiterhin umfassend eine Steuereinheit (300), die so konfiguriert ist, dass sie den ersten oder zweiten Betriebsmodus aktiviert.

4. Vitalzeichenüberwachungssystem nach Anspruch 1, wobei die Kohärenzverzerrungseinheit (200) so konfiguriert ist, dass sie einen der mindestens einen Laserlichtquelle (110) zugeführten Eingangsstrom steuert, um die Wellenfront des Laserlichts aus der mindestens einen Laserlichtquelle (110) in dem ersten Betriebsmodus zu verzerren.

5. Vitalzeichenüberwachungssystem nach Anspruch 1, wobei die Kohärenzverzerrungseinheit (200) einen Phasenmodulator (130) umfasst, der so konfiguriert ist, dass er eine Phase der Wellenfront des Laserlichts aus der mindestens einen Laserlichtquelle (110) verzerrt.

6. Vitalzeichenüberwachungssystem nach Anspruch 1, wobei die Kohärenzverzerrungseinheit (200) einen elektrisch gesteuerten Diffusor (130) umfasst, um die Wellenfront des Laserlichts aus der mindestens einen Laserlichtquelle (110) räumlich zu verzerren.

7. Vitalzeichenüberwachungssystem nach Anspruch 1, wobei die Kohärenzverzerrungseinheit (200) einen räumlichen Modulator (130) für Licht umfasst, um die Wellenfront des Laserlichts aus der mindestens einen Laserlichtquelle (110) räumlich zu verzerren.

8. Vitalzeichenüberwachungssystem nach Anspruch 1, wobei die Kohärenzverzerrungseinheit (200) eine elektrochrome oder Flüssigkristalleinheit (130) umfasst, um die Wellenfront des Laserlichts aus der mindestens einen Laserlichtquelle (110) durch Verändern der Diffusität räumlich zu verzerren.

9. Vitalzeichenüberwachungssystem nach Anspruch 1, wobei die Kohärenzverzerrungseinheit (200) einen Wellenlängenmodulator (130) umfasst, der so konfiguriert ist, dass er die Wellenlänge des Lichts verändert, um die Wellenfront des Lichts aus der mindestens einen Laserlichtquelle (110) zu verzerren.

10. Vitalzeichenüberwachungssystem nach Anspruch 1, wobei die mindestens eine Laserlichtquelle (110) einen Festkörperlaser mit abgestufter, ultradünner Kavität umfasst.

11. Verfahren zur Überwachung von Vitalzeichen eines Benutzers, das die folgenden Schritte umfasst, wonach:
- Vitalzeichen eines Benutzers durch mindestens einen optischen Vitalzeichensensor (100) gemessen oder ermittelt werden, wobei der mindestens eine optische Vitalzeichensensor (100) mindestens eine Laserlichtquelle (110), die so eingerichtet ist, dass sie Laserlicht erzeugt, das auf eine Haut (1000) eines Benutzers gerichtet ist, sowie min-destens eine Photodetektoreinheit (120) umfasst, die so konfiguriert ist, dass sie Licht, das für eine Absorption oder Reflexion des Laserlichts aus der mindestens einen Lichtquelle (100) bezeichnend ist, in oder auf der Haut (1000) des Benutzers detektiert, und
- in einem ersten Betriebsmodus eine Wellenfront des Laserlichts aus der mindestens einen Laserlichtquelle (110) so verzerrt wird, dass nicht-kohärentes Licht auf die Haut (1000) des Benutzers gerichtet wird, und
- in einem zweiten Betriebsmodus die Verzerrung der Wellenfront des Laserlichts so deaktiviert wird, dass kohärentes Licht auf die Haut (1000) des Benutzers gerichtet wird, und
- das Ausgangssignal des mindestens einen optischen Vitalzeichensensors (100) analysiert wird, wobei in dem ersten Betriebsmodus der Analysierungsschritt so vorgesehen ist, dass die Ausgabe des mindestens einen Photodetektors (120) analysiert wird, um eine Herzfrequenz des Benutzers zu ermitteln, wobei in einem zweiten Betriebsmodus der Analysierungsschritt so vorgesehen ist, dass die Ausgabe des mindestens einen Photodetektors (120) analysiert wird, um Mikrozirkulationsparameter des Benutzers zu ermitteln.

12. Computerprogrammprodukt, umfassend einen computerlesbaren Speicher, der Computerprogrammcodemittel speichert, um zu bewirken, dass ein Prozessor Schritte des Überwachens von Vitalzeichen eines Benutzers nach Anspruch 11 ausführt.

## Revendications

1. Système de surveillance des signes vitaux, comprenant :
- au moins un capteur optique des signes vitaux (100) configuré pour mesurer ou déterminer des signes vitaux d'un utilisateur et pour émettre un signal de sortie, dans lequel ledit au moins un capteur optique des signes vitaux (100) comprend au moins une source de lumière laser (110) configurée pour générer une lumière laser, qui est dirigée vers une peau (1000) de l'utilisateur, et au moins une unité de photodétecteur (120) configurée pour détecter une lumière qui est indicative d'une absorption ou d'une réflexion de la lumière laser à partir de l'au moins une source de lumière (100) dans ou depuis la peau (1000) de l'utilisateur,
- une unité de distorsion de cohérence (200) dans un premier mode opérationnel configuré pour distordre un front d'ondes de la lumière laser de l'au moins une source de lumière laser (110) de telle sorte que la lumière non cohérente soit dirigée vers la peau (1000) de l'utilisateur et dans un deuxième mode opérationnel, configuré pour désactiver la distorsion du front d'onde de la lumière laser de telle sorte que la lumière cohérente soit dirigée vers la peau (1000) de l'utilisateur, et
- une unité d'analyse (400) ayant une unité d'analyse de la fréquence cardiaque (410) et une unité d'analyse de la microcirculation (420) et étant configurée pour analyser le signal de sortie de l'au moins un capteur optique des signes vitaux (100), dans lequel dans le premier mode opérationnel, l'unité d'analyse de la fréquence cardiaque (410) est configurée pour analyser l'émission de l'au moins un photodétecteur (120) pour déterminer une fréquence cardiaque de l'utilisateur, dans lequel dans un deuxième mode opérationnel, l'unité d'analyse de la microcirculation (420) est configurée pour analyser la sortie de l'au moins un photodétecteur (120) pour déterminer des paramètres de microcirculation de l'utilisateur.

2. Système de surveillance des signes vitaux selon la revendication 1, dans lequel l'au moins un capteur optique des signes vitaux (100) est un capteur photopléthysmographique.

3. Système de surveillance des signes vitaux selon la revendication 2, comprenant en outre une unité de commande (300) configurée pour activer le premier ou deuxième mode opérationnel.

4. Système de surveillance des signes vitaux selon la revendication 1, dans lequel l'unité de distorsion de cohérence (200) est configurée pour commander un courant d'entrée fourni à l'au moins une source de laser (110) afin de distordre le front d'onde de la lumière laser de l'au moins une source de lumière laser (110) dans le premier mode opérationnel.

5. Système de surveillance des signes vitaux selon la revendication 1, dans lequel l'unité de distorsion de cohérence (200) comprend un modulateur de phase (130) configuré pour distordre une phase du front d'onde de la lumière laser de l'au moins une source de lumière laser (110).

6. Système de surveillance des signes vitaux selon la revendication 1, dans lequel l'unité de distorsion de cohérence (200) comprend un diffuseur (130) commandé électriquement pour distordre spatialement le front d'onde de la lumière laser de l'au moins une source de lumière laser (110).

7. Système de surveillance des signes vitaux selon la revendication 1, dans lequel l'unité de distorsion de cohérence (200) comprend un modulateur spatial de lumière (130) pour distordre spatialement le front d'onde de la lumière laser de l'au moins une source de lumière laser (110).

8. Système de surveillance des signes vitaux selon la revendication 1, dans lequel l'unité de distorsion de cohérence (200) comprend une unité électro-chromique ou de cristal liquide (130) pour distordre spatialement le front d'onde de la lumière laser de l'au moins une source de lumière laser (110) en modifiant la diffusivité.

9. Système de surveillance des signes vitaux selon la revendication 1, dans lequel l'unité de distorsion de cohérence (200) comprend un modulateur de longueur d'ondes (130) configuré pour changer la longueur d'ondes de la lumière pour distordre le front d'ondes de la lumière de l'au moins une source de lumière laser (110).

10. Système de surveillance des signes vitaux selon la revendication 1, dans lequel l'au moins une source de lumière laser (110) comprend un laser à état solide à cavité ultra-fine échelonné.

11. Procédé de surveillance des signes vitaux, comprenant les étapes consistant à :
- mesurer ou déterminer des signes vitaux d'un utilisateur par au moins un capteur optique des signes vitaux (100), dans lequel ledit au moins un capteur optique des signes vitaux (100) comprend au moins une source de lumière laser (110) configurée pour générer une lumière laser, qui est dirigée vers une peau (1000) d'un utilisateur, et au moins une unité de photodétecteur (120) configurée pour détecter une lumière qui est indicative d'une absorption ou d'une réflexion de la lumière laser à partir de l'au moins une source de lumière (100) dans ou depuis la peau (1000) de l'utilisateur, et
- dans un premier mode opérationnel, distordre un front d'ondes de la lumière laser de l'au moins une source de lumière laser (110) de telle sorte que la lumière non cohérente soit dirigée vers la peau (1000) de l'utilisateur, et
- dans un deuxième mode opérationnel, désactiver la distorsion du front d'onde de la lumière laser de telle sorte que la lumière cohérente soit dirigée vers la peau (1000) de l'utilisateur, et
- analyser le signal de sortie de l'au moins capteur optique des signes vitaux (100), dans lequel dans le premier mode opérationnel, l'étape d'analyse est adaptée pour analyser la sortie de l'au moins un photodétecteur (120) pour déterminer une fréquence cardiaque de l'utilisateur, dans lequel dans un deuxième mode opérationnel, l'étape d'analyse est adaptée pour analyser la sortie de l'au moins un photodétecteur (120) pour déterminer des paramètres de microcirculation de l'utilisateur.

12. Produit de programme informatique, comprenant une mémoire pouvant être lue par un ordinateur stockant des moyens de codage d'un programme informatique pour faire réaliser par un processeur, des étapes de la surveillance des signes vitaux d'un utilisateur selon la revendication 11.
